(19) Europäisches Patentamt
European Patent Office
Office européen des brevets

(11) Veröffentlichungsnummer: **0 421 270 A2**

(12) **EUROPÄISCHE PATENTANMELDUNG**

(21) Anmeldenummer: **90118533.0**

(22) Anmeldetag: **27.09.90**

(51) Int. Cl.⁵: **C07D 249/02**, C07D 249/08, A01N 43/647, A01N 43/653

(30) Priorität: **06.10.89 US 418090**

(43) Veröffentlichungstag der Anmeldung: **10.04.91 Patentblatt 91/15**

(84) Benannte Vertragsstaaten: **BE CH DE FR GB IT LI NL**

(71) Anmelder: **BASF Aktiengesellschaft**
**Carl-Bosch-Strasse 38**
**W-6700 Ludwigshafen(DE)**

(72) Erfinder: **Seele, Rainer, Dr.**
**Leiblstrasse 3**
**W-6701 Fussgoenheim(DE)**
Erfinder: **Karbach, Stefan, Dr.**
**Grundwiesenweg 44**
**W-6730 Neustadt(DE)**
Erfinder: **Kober, Reiner, Dr.**
**Im Schlittweg**
**W-6701 Fussgoenheim(DE)**
Erfinder: **Zipplies, Matthias, Dr.**
**Kastanienweg 1**
**W-6945 Hirschberg(DE)**
Erfinder: **Sauter, Hubert, Dr.**
**Neckarpromenade 20**
**W-6800 Mannheim 1(DE)**
Erfinder: **Moore, Barbara Auxier**
**Route 4, Box 162**
**Pittsboro, N.C. 27312(US)**
Erfinder: **Carlson, Dale R., Dr.**
**107 King Street**
**Hillsborough, N.C. 27278(US)**
Erfinder: **Zorner, Paul Steffen, Dr.**
**19 Sanderling Court**
**Durham, N.C. 27713(US)**
Erfinder: **Westphalen, Karl-Otto, Dr.**
**Mausbergweg 58**
**W-6720 Speyer(DE)**
Erfinder: **Wuerzer, Bruno, Dr.**
**Ruedigerstrasse 13**
**W-6701 Otterstadt(DE)**

(54) **N-Aminotriazolderivate.**

(57) N-Aminotriazolderivate der allgemeinen Formel Ia und Ib,

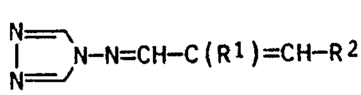

Ia

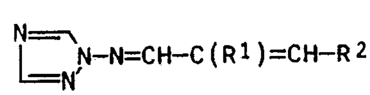

Ib

in der die Substituenten folgende Bedeutung haben:
$R^1$, $R^2$ $C_1$-$C_8$-Alkyl; $C_3$-$C_8$-Cycloalkyl; $C_5$-$C_8$-Cycloalkenyl; Tetrahydropyranyl; Norbornyl;
Phenyl, Biphenyl, Naphthyl oder Pyridyl, wobei die aromatischen Reste ein bis fünf Halogenatome und/oder ein bis drei der folgenden Gruppen tragen können: Hydroxy, Phenoxy, Nitro, Amino, Cyano, $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio
sowie deren Umwelt- und Kulturpflanzen-verträgliche Salze.

EP 0 421 270 A2

# N-AMINOTRIAZOLDERIVATE

Die vorliegende Erfindung betrifft N-Aminotriazolderivate der allgemeinen Formel Ia und Ib,

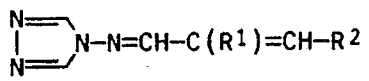

Ia

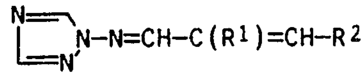

Ib

in der die Substituenten folgende Bedeutung haben:

$R^1$, $R^2$ $C_1$-$C_8$-Alkyl; $C_3$-$C_8$-Cycloalkyl; $C_5$-$C_8$-Cycloalkenyl; Tetrahydropyranyl; Norbornyl; Phenyl, Biphenyl, Naphthyl oder Pyridyl, wobei die aromatischen Reste ein bis fünf Halogenatome und/oder ein bis drei der folgenden Gruppen tragen können: Hydroxy, Phenoxy, Nitro, Amino, Cyano, $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio

sowie deren Umwelt- und Kulturpflanzen-verträgliche Salze.

Außerdem betrifft die Erfindung Verfahren zur Herstellung der Verbindungen Ia und Ib, herbizide Mittel, die N-Aminotriazolderivate als Synergisten enthalten sowie Verfahren zur selektiven Bekämpfung von unerwünschtem Pflanzenwuchs mit diesen herbiziden Mitteln.

Aus der Literatur sind N-Aminotriazolderivate als Fungizide bekannt (EP-A 283 245).

Herbizide Wirkstoffe aus der Gruppe der Benzothiadiazinderivate der Formel II

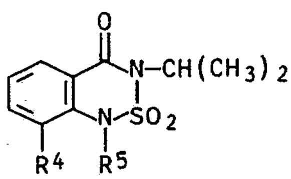

II

in der die Substituenten folgende Bedeutung haben: $R^4$ Wasserstoff; Halogen; $C_1$-$C_4$-Alkyl oder $C_1$-$C_4$-Alkoxy und

$R^5$ Wasserstoff oder Cyano

oder deren Umwelt- und Kulturpflanzen-verträglichen Salze dienen der Bekämpfung unerwünschter Pflanzen (DE-A 1 542 836, USA 4 158 559, US-A 3 920 441, DE-A 1 918 946, USA 3 954 437, US-A 4 464 195).

Der Erfindung lagen Verbindungen als Aufgabe zugrunde, welche die Wirkung der obengenannten Herbizide gegen unerwünschte Pflanzen überadditiv steigern, ohne daß die Kulturpflanzenverträglichkeit verlorengeht. Solche Verbindungen werden im allgemeinen als "Synergisten" bezeichnet.

Entsprechend dieser Aufgabe wurden die eingangs definierten N-Aminotriazolderivate Ia und Ib gefunden.

Weiterhin wurden Verfahren zur Bekämpfung unerwünschten Pflanzenwuchses durch Anwendung dieser N-Aminotriazole Ia und/oder Ib in Verbindung mit den Herbiziden aus der Gruppe der Benzothiadiazine II und entsprechend Mittel zur Bekämpfung unerwünschten Pflanzenwuchses gefunden.

Die N-Aminotriazole Ia und Ib enthalten eine dreifachsubstituierte Doppelbindung. Sie können demnach in E- und Z-Konfiguration vorliegen.

Die erfindungsgemäßen N-Aminotriazolderivate Ia und Ib sind auf verschiedenen Wegen zugänglich.

Man erhält sie bevorzugt, indem man ein N-Aminotriazol IIIa bzw. IIIb in an sich bekannter Weise in einem inerten organischen Lösungsmittel mit einem entsprechenden Acroleinderivat IV gemäß dem folgenden Schema umsetzt.

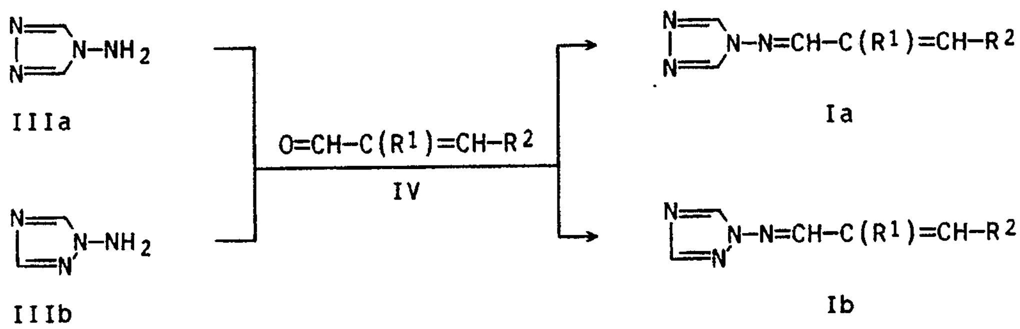

Diese Umsetzung kann kontinuierlich oder diskontinuierlich, bei Normaldruck oder unter Drucken bis 30 bar, vorzugsweise bei Normaldruck bis 5 bar und Temperaturen von 20 °C bis 150 °C, vorzugsweise 50 °C bis 100 °C, durchgeführt werden.

Als Lösungsmittel eignen sich beispielsweise Nitrile wie Acetonitril und Propionitril, Alkohole wie Methanol, Ethanol, iso-Propanol, n-Butanol und iso-Butanol, Ether wie Diethylether, Diisopropylether, tert.-Butylmethylether, Dimethoxyethan, Dioxan und Tetrahydrofuran sowie vorzugsweise Kohlenwasserstoffe und Halogenkohlenwasserstoffe wie Pentan, Hexan, Cyclohexan, Toluol, Methylenchlorid, Chloroform, Tetrachlorkohlenstoff, Dichlorethan und Chlorbenzol.

Die für die Umsetzung benötigten Acroleinderivate der Formel III sind bekannt oder lassen sich nach den üblichen Verfahren zur Aldehydsynthese herstellen (Houben-Weyl, Bd. E3, 1983).

Im Hinblick auf die bestimmungsgemäße Verwendung der N-Aminotriazolderivate Ia und Ib als synergistische Mittel kommen als Substituenten $R^1$ und $R^2$ unabhängig voneinander vorzugsweise folgende Reste in Betracht:

Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, Alkylthiogruppe bevorzugt in 1-, 2- oder 3-Position substituiert sind, insbesondere 2-Ethylthiopropyl; insbesondere unverzweigtes $C_1$-$C_6$-Alkyl;

Cycloalkyl wie Cyclopropyl, Cyclobutyl, Cyclopentyl, Cyclohexyl, Cycloheptyl und Cyclooctyl, insbesondere Cyclopentyl und Cyclohexyl;

Cycloalkenyl wie Cyclopentenyl, Cyclohexenyl, Cycloheptenyl und Cyclooctenyl, insbesondere Cyclohexenyl;

Tetrahydropyranyl; Norbornyl;

Phenyl, Biphenyl, Naphthyl oder Pyridyl, wobei die aromatischen Reste ein bis fünf Halogenatome wie Fluor, Chlor, Brom und Iod, insbesondere Fluor, Chlor und Brom;

und/oder ein bis drei der folgenden Gruppen tragen können: Hydroxy; Phenoxy, Nitro; Amino; Cyano; Alkyl wie vorstehend genannt mit ein bis vier Kohlenstoffatomen, insbesondere Methyl;

Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Trifluormethyl;

Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy, Ethoxy, 1-Methylethoxy und 1,1-Dimethylethoxy, insbesondere Methoxy;

Halogenalkoxy wie Trifluormethoxy, Chlor-difluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy;

und/oder Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio;

sowie deren Umwelt- und Kulturpflanzen-verträglichen Salze.

Beispiele für insbesondere bevorzugte Verbindungen Ia und Ib sind in den folgenden Tabellen A und B aufgeführt.

Tabelle A

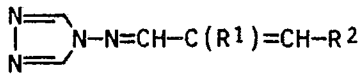

Ia

| $R^1$ | $R^2$ |
|---|---|
| $4\text{-F-}C_6H_4$ | $2\text{-Cl-}C_6H_4$ |
| $4\text{-F-}C_6H_4$ | $3\text{-Cl-}C_6H_4$ |
| $4\text{-F-}C_6H_4$ | $4\text{-Cl-}C_6H_4$ |
| $4\text{-F-}C_6H_4$ | $2,4\text{-Cl,Cl-}C_6H_3$ |
| $4\text{-F-}C_6H_4$ | $2\text{-F-}C_6H_4$ |
| $4\text{-F-}C_6H_4$ | $4\text{-F-}C_6H_4$ |
| $4\text{-F-}C_6H_4$ | $2\text{-Br-}C_6H_4$ |
| $4\text{-F-}C_6H_4$ | $2\text{-}OCH_3\text{-}C_6H_4$ |
| $4\text{-F-}C_6H_4$ | $2\text{-}CH_3\text{-}C_6H_4$ |
| $4\text{-F-}C_6H_4$ | $4\text{-}NO_2\text{-}C_6H_4$ |
| $4\text{-F-}C_6H_4$ | $4\text{-}NH_2\text{-}C_6H_4$ |
| $4\text{-F-}C_6H_4$ | $2\text{-}CF_3\text{-}C_6H_4$ |
| $4\text{-F-}C_6H_4$ | $4\text{-}CF_3\text{-}C_6H_4$ |
| $4\text{-F-}C_6H_4$ | $C_6H_5\text{-O-}C_6H_4$ |
| $4\text{-F-}C_6H_4$ | 2-Naphthyl |
| $4\text{-F-}C_6H_4$ | 3-Pyridyl |
| $4\text{-F-}C_6H_4$ | 4-Biphenyl |
| $4\text{-F-}C_6H_4$ | Cyclohexyl |
| $4\text{-F-}C_6H_4$ | 3-Cyclohexenyl |
| $4\text{-F-}C_6H_4$ | 4-Tetrahydropyranyl |
| $C_6H_5$ | $C_6H_5$ |
| $C_6H_5$ | $2\text{-Cl-}C_6H_4$ |
| $C_6H_5$ | $4\text{-Cl-}C_6H_4$ |
| $C_6H_5$ | $2,4\text{-Cl,Cl-}C_6H_3$ |
| $C_6H_5$ | $2\text{-F-}C_6H_4$ |
| $C_6H_5$ | $4\text{-F-}C_6H_4$ |
| $C_6H_5$ | $2\text{-}OCH_3\text{-}C_6H_4$ |

Tabelle A (Fortsetzung)

| $R^1$ | $R^2$ |
|---|---|
| $C_6H_5$ | $2-CF_3-C_6H_4$ |
| $2-Cl-C_6H_4$ | $4-Cl-C_6H_4$ |
| $2-Cl-C_6H_4$ | $2,4-Cl,Cl-C_6H_3$ |
| $2-Cl-C_6H_4$ | $4-F-C_6H_4$ |
| $2-Cl-C_6H_4$ | $2-Br-C_6H_4$ |
| $2-Cl-C_6H_4$ | $2-CF3-C_6H_4$ |
| $4-Cl-C_6H_4$ | $2-Cl-C_6H_4$ |
| $4-Cl-C_6H_4$ | $4-Cl-C_6H_4$ |
| $4-Cl-C_6H_4$ | $2-F-C_6H_4$ |
| $4-Cl-C_6H_4$ | $4-F-C_6H_4$ |
| $4-Cl-C_6H_4$ | $2-Br-C_6H_4$ |
| $4-Cl-C_6H_4$ | $2-OCH_3-C_6H_4$ |
| $4-Cl-C_6H_4$ | $2-CH_3-C_6H_4$ |
| $4-Cl-C_6H_4$ | $2-CF_3-C_6H_4$ |
| $2,4-Cl,Cl-C_6H_3$ | $2-Cl-C_6H_4$ |
| $2,4-Cl,Cl-C_6H_3$ | $4-Cl-C_6H_4$ |
| $2,4-Cl,Cl-C_6H_3$ | $2-F-C_6H_4$ |
| $2,4-Cl,Cl-C_6H_3$ | $2-F-C_6H_4$ |
| $2,4-Cl,Cl-C_6H_3$ | $4-F-C_6H_4$ |
| $2,4-Cl,Cl-C_6H_3$ | $2-CF_3-C_6H_4$ |
| $2,4-Cl,Cl-C_6H_3$ | $2-OCH_3-C_6H_4$ |
| $2,4-Cl,Cl-C_6H_3$ | $2-CH_3-C_6H_4$ |
| $4-OCH_3-C_6H_4$ | $2-Cl-C_6H_4$ |
| $4-OCH_3-C_6H_4$ | $4-Cl-C_6H_4$ |
| 4-tert.-$C_4H_9-C_6H_4$ | $2-Cl-C_6H_4$ |
| 4-tert.-$C_4H_9-C_6H_4$ | $2-F-C_6H_4$ |
| 4-tert.-$C_4H_9-C_6H_4$ | $4-F-C_6H_4$ |
| 2-Naphthyl | $2-Cl-C_6H_4$ |
| 2-Naphthyl | $4-Cl-C_6H_4$ |
| tert.-$C_4H_9$ | $2-Cl-C_6H_4$ |
| tert.-$C_4H_9$ | $4-Cl-C_6H_4$ |
| tert.-$C_4H_9$ | $2-F-C_6H_4$ |
| tert.-$C_4H_9$ | $4-F-C_6H_4$ |
| tert.-$C_4H_9$ | $2,4-Cl,Cl-C_6H_3$ |
| tert.-$C_4H_9$ | $2-CF_3-C_6H_4$ |
| Cyclohexyl | $2-Cl-C_6H_4$ |
| Cyclohexyl | $4-Cl-C_6H_4$ |

Tabelle A (Fortsetzung)

| $R^1$ | $R^2$ |
|---|---|
| Cyclohexyl | 2,4-Cl,Cl-$C_6H_3$ |
| Cyclohexyl | 2-F-$C_6H_4$ |
| 4-tetrahydropyranyl | 2-Cl-$C_6H_4$ |
| 4-tetrahydropyranyl | 4-Cl-$C_6H_4$ |
| 4-F-$C_6H_4$ | Norbornyl |

Tabelle B

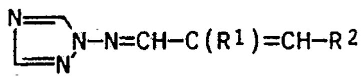

Ib

| $R^1$ | $R^2$ |
|---|---|
| 4-F-$C_6H_4$ | 2-Cl-$C_6H_4$ |
| 4-F-$C_6H_4$ | 4-Cl-$C_6H_4$ |
| 4-F-$C_6H_4$ | 2-Br-$C_6H_4$ |
| 4-F-$C_6H_4$ | 2-$CH_3$-$C_6H_4$ |
| 4-F-$C_6H_4$ | 2-$CF_3$-$C_6H_4$ |
| $C_6H_5$ | 2-Cl-$C_6H_4$ |
| 2-Cl-$C_6H_4$ | 4-F-$C_6H_4$ |
| 4-Cl-$C_6H_4$ | 2-$CF_3$-$C_6H_4$ |
| tert.-$C_4H_9$ | 2-Cl-$C_6H_4$ |
| tert.-$C_4H_9$ | 4-Cl-$C_6H_4$ |
| tert.-$C_4H_9$ | 4-F-$C_6H_4$ |
| tert.-$C_4H_9$ | 2,4-Cl,Cl-$C_6H_3$ |
| Cyclohexyl | 4-Cl-$C_6H_4$ |
| Cyclohexyl | 2,4-Cl,Cl-$C_6H_3$ |
| Cyclohexyl | 4-F-$C_6H_4$ |

Spezielle Beispiele für herbizide Benzothiadiazinderivate der Formel II, deren Wirksamkeit durch die Synergistischen N-Aminotriazolderivate Ia und Ib verbessert werden kann, sind in der folgenden Tabelle aufgeführt.

Tabelle C

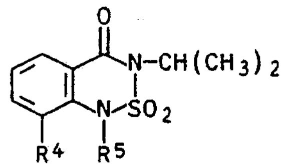

II

| Nr. | $R^4$ | $R^5$ | Literatur |
|---|---|---|---|
| II.001 | H | H | DE-A 1 542 836 |
| II.002 | Cl | H | DE-A 2 444 383 |
| II.003 | F | H | DE-A 2 444 383 |
| II.004 | $CH_3$ | H | DE-A 2 443 901 |
| II.005 | H | Na | DE-A 1 542 836 |
| II.006 | Cl | Na | DE-A 2 444 383 |
| II.007 | F | Na | DE-A 2 444 383 |
| II.008 | $CH_3$ | Na | DE-A 2 443 901 |
| II.009 | Cl | CN | DE-A 2 656 289 |
| II.010 | F | CN | DE-A 2 656 289 |
| II.011 | $CH_3$ | CN | DE-A 2 656 289 |
| II.012 | H | CN | DE-A 2 656 289 |

Für herbizide Benzothiadiazinderivate der Formel II werden unterschiedliche Mengen einer synergistisch wirkenden Verbindung benötigt, wenn das herbizid in verschiedenen Kulturen eingesetzt wird. Die Mengenverhältnisse sind in weiten Bereichen variabel. Sie sind außerdem abhängig von der Struktur des Herbizids II und der jeweiligen Zielkultur.

Geeignete Anteilsverhältnisse von Synergist zu herbizidem Wirkstoff liegen bei 10:1 bis 0,01:1, vorzugsweise bei 6:1 bis 0,05:1 und insbesondere bei 4:1 bis 0,1:1 Gew.-Teile.

Herbizide Wirkstoffe und synergistisch wirkende Verbindungen können gemeinsam oder getrennt nach dem Auflaufen auf die Blätter und Sprosse der Kulturpflanzen und der unerwünschten Pflanzen ausgebracht werden. Bevorzugt wird das synergistisch wirkende Mittel gleichzeitig mit dem herbiziden Wirkstoff ausgebracht. Auch eine getrennte Ausbringung, wobei der Synergist und der herbizide Wirkstoff gleichzeitig oder zeitlich getrennt nacheinander auf das Feld gebracht werden, ist möglich. Herbizider Wirk stoff und Synergist können hierbei als Spritzmittel in suspendierbarer, emulgierbarer oder löslicher Form gemeinsam oder getrennt formuliert vorliegen.

Die erforderlichen Aufwandmengen an reiner Wirkstoffmenge, d.h. ohne Formulierungshilfsmittel ist außerdem abhängig von der Zusammensetzung des Pflanzenbestandes, vom Entwicklungsstadium der Pflanzen, von den klimatischen Verhältnissen am Einsatzort sowie von der Anwendungstechnik. Im allgemeinen betragen die Aufwandmengen 0,25 bis 5 kg, vorzugsweise 0,5 bis 2,5 kg Wirkstoff/ha.

Als Kulturen, in denen die erfindungsgemäßen herbiziden und pflanzenschützenden Mittel angewandt werden können, kommen im wesentlichen diejenigen in Betracht, in denen auch die Einzelwirkstoffe der Mischung eingesetzt werden können. Im Falle von Benzothiadiazincnderivate der Formel II enthaltenden Mittel sind dies beispielsweise Getreide, Erdnüsse, Reis, Soja, Mais, Kultursorghum und Erbsen.

Von Bedeutung ist ferner die Ausbringungstechnik. Werden die neuen Mittel zum Zwecke der Bekämpfung unerwünschter Pflanzen bei Kulturpflanzen, die eine ungenügende Verträglichkeit aufweisen, eingesetzt, so wird nach speziellen Methoden die Ausbringung so gelenkt, daß die Blätter der Kulturpflanzen möglichst wenig mit den Mitteln in Berührung kommen, während die dazwischen oder darunter wachsenden unerwünschten Pflanzen oder die freie Bodenfläche von den Mitteln getroffen werden (Unterblattspritzung, post-directed, lay-by).

Die erfindungsgemäßen herbiziden und pflanzenschützenden Mittel können zusätzlich mit zahlreichen Vertretern weiterer herbizider oder wachstumsregulierender Wirkstoffgruppen gemischt und gemeinsam angebracht werden.

Neben der synergistischen Wirkung bei der gemeinsamen Anwendung mit Benzothiadiazinen der

Formel II können die N-Aminotriazolderivate Ia und Ib auch bei folgenden Herbiziden als Synergisten eingesetzt werden (Handelsnamen in Klammern)
- 5-Amino-4-chlor-2-phenylpyridazin-3(2H)-on (Pyrazon)
- 4-Chlor-5-methylamino-2-(trifluormethylphenyl)-3-(2H)-pyridazin-3(2H)-on (Monometfluorazon)
- 3-(3-Chlor-4-methylphenyl)-1,1-dimethylharnstoff (Chlortoluron)
- 3-(4-Bromphenyl)-1-methoxy-1-methylharnstoff (Metobromuron)
- 3-(4-Isopropylphenyl)-1,1-dimethylharnstoff (Isoproturon)
- 3-(3,4-Dichlorphenyl)1-methoxy-1-methylharnstoff (Linuron)
- 3-(3,4-Dichlorphenyl)-1,1-dimethylharnstoff (Diuron)
- 3-(2-Benzothiazolyl)1,3-dimethylharnstoff (Methabenzthiazuron)
- 1,1-Dimethyl-3-(3-trifluormethylphenyl)-harnstoff (Fluometuron)
- 2-[3-(4-Methoxy-6-methyl-1,3,5-triazin-2-yl)aminocarbonylaminosulfonyl]benzsäure)methylester (Metsulfuron-methyl)
- 2-[3-(4,6-dimethoxypyrimidin-2-yl)aminocarbonylaminosulfonyl]benzoesäuremethylester (Bensulfuron-methyl)
- 2-[3-(4-Chlor-6-methoxypyrimidin-2-yl)aminocarbonylaminosulfonyl]benzoesäureethylester (Chlorimuron)
- 2-[3-(4-Methoxy-6-methyl-1,3,5-triazin-2-yl)-3-methylaminocarbonyl-aminosulfonyl]benzoesäuremethylester
- 3-(4,6-Dimethoxy-1,3,5-triazin-2-yl)-1-[2-(2-methoxyethoxy)phenylsulfonyl]harnstoff (Cinosulfuron)
- 2-[3-(4,6-bis(difluormethoxy)pyrimidin-2-yl)-aminocarbonylaminosulfonyl]benzoesäuremethylester-(Primisulfuron)
- 2-(2-Chlorethoxy)N-[[(4-methoxy-6-methyl-1,3,5-triazin-2-yl)aminocarbonyl]benzolsulfonsäureamid (Triasulfuron)
- 2[[(4,6-Dimethoxypyrimidin-2-yl)aminocarbonyl]aminosulfonyl]-3-pyridincarbonsäure-N,N-dimethylamid
- S-(4-Chlorbenzyl)-N,N-diethylthiocarbamat (Benthiocarb)
- S-Benzyl-N,N-dipropylthiocarbamat (Prosulfocarb)
- S-Ethyl-N,N,-di-iso-butylthiocarbamat (Butylat)
- S-EthylN,N-di-n-propylthiocarbamat (EPTC)
- 3-(Methoxycarbonylamino)phenyl-N-(3-methylphenyl)carbamat (Phenmedipham)
- 3-(Ethoxycarbonylamino)phenyl-N-phenylcarbamat (Desmedipham)
- Isopropyl-N-(3-chlorphenyl)-carbamat (Chloropropham)
- 2,6-Dinitro-N,N-dipropyl-4-trifluormethylanilin (Trifluralin)
- 3,4-Dimethyl-2,6-Dinitro-N-1-ethylpropyl-anilin (Pendimenthalin)
- 4-Amino-3-methyl-6-phenyl-1,2,4-triazin-5(4H)-on (Metamitron)
- 4-Amino-6-tert.butyl-3-methylthio-1,2,4-triazin-5(4H)-on (Metribuzin) 2-(2-Chlor-4-ethylamino-1,3,5-triazin-yl-amino)-2-methylpropionitril
- (Cyanazin) 2-Chlor-4-ethylamino-6-iso-propylamino-1,3,5-triazin (Atrazin)
- 2-Chlor-4-ethylamino-6-tert.butylamino-1,3,5-triazin
- (Terbutylazin) 3-Chlor-4-chromethyl-1-(3-trifluormethylphenyl)pyrrolidin-2-on
- (Fluorochloridin) 2-Chlor-6-nitro-3-phenoxyanilin (Aclonifen)
- 3,6-Dichlor-2-methoxybenzoesäure (Dicamba)
- 2,5-Dichlor-3-aminobenzoesäure (Amiben)
- 2,4-Dichlorphenoxyessigsäure (2,4-D)
- 2-(2,4-Dichlorphenoxy)propionsäure (Dichloprop)
- 2-(4-Chlor-2-methylphenoxy)propionsäure (Mecoprop)
- 2-[4-(2,4-Dichlorphenoxy)-phenoxy]propionsäuremethylester (Diclofop-methyl)
- 2-[4-(6-Chlor-2-benzoxazolyloxy)phenoxy]propionsäureethylester (Fenoxaprop-ethyl)
- 2-[4-(6-Chlor-2-chinoxanyloxy)phenoxy]propionsäureethylester (Quizalafop-ethyl)
- 2-[4-(3-Chlor-5-trifluormethyl-2-pyridyloxy)phenoxy]propionsäuremethylester (Haloxyfop-methyl)
- 2-[4-(5-Trifluormethyl-2-pyridyloxy)phenoxy]propionsäurebutylester (Fluazifop-bentyl)

4-Amino-3,5-dichlor-6-fluor-2-pyridinyloxyessigsäure, dessen Salze und Alkylester, z.B. 1-Methylheptylester (Fluroxypyr)
- 7-Chlor-3-methyldinolin-8-carbonsäure (Quinmerac)
- 3,7-Dichlorchinolin-8-carbon-säure (Quinclorac)
- N-(2,4-Difluorphenyl)-2-(3-trifluormethylphenoxy)-3-pyridincarbonsäureamid (Diflufanican)
- exo-1-Methyl-4-(1-methylethyl)-2-(2-methylphenylmethoxy)-7-oxabicyclo(2.2.1)heptan (Cinmethlin)
- 2-(2-Chlorphenyl)methyl-4,4-dimethyl-3-is-oxxazolidinon (Clomazon)
- 5-Methylamino-2-phenyl-4-(3-trifluormethylphenyl)furan-3(2H)-on (Flurtamon)
- 2-[4,5-Dihydro-4-methyl-4-(1-methylethyl)-5-oxo-1H-imidazol-2-yl]-5-ethyl-3-pyridin-carbonsäure

(Imazethapyr)

- 2-[4,5-Dihydro-4-methyl-4-(1-methylethyl)-5-oxo-1H-imidazol-2-yl)-3-chinolincarbonsäure (Imazaquin)
- 4-Chlor-2-oxobenzothiazolin-3-ylessigsäure (Benazolin)
- 2-Phenyl-3,1-benzoxazin-4-on
- 5-Fluor-2-phenyl-4H-3,1-benzoxazin-4-on (Fluorobentranil)
- 3′,4′-Dichlorpropionanilid (Propanil)
- 5-[2-Chlor-4-(trifluormethyl)phenoxyx]-2-nitrobenzoesäure-natriumsalz (Acifluorfen)
- 5-(2,4-Dichlorphenoxy)-2-nitrobenzoesäure-methylester (Bifenox)
- 5-(2-Chlor-4-trifluormethylphenoxy)-2-nitro-N-methansulfonylbenzoesäureamid (Fomesafen)
- 3,5-Dibrom-4-hydroxybenzonitril (Bromoxynil)
- 3,5-Dijod-4-hydroxybenzonitril (Ioxynil)

Außerdem ist es nützlich, die erfindungsgemäßen Mischungen auch noch mit weiteren Pflanzenschutzmitteln gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralstofflösungen, welche zur Behebung von Ernährungs- und Spurenelementmängeln eingesetzt werden.

Die erfindungsgemäßen Mittel bzw. bei getrennter Ausbringung die herbiziden Wirkstoffe oder der Synergist werden beispielsweise in Form von direkt versprühbaren Lösungen, Pulvern, Suspensionen, auch hochprozentigen wäßrigen, öligen oder sonstigen Suspensionen, oder Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken.

Die Formulierungen enthalten zwischen 0,1 und 95, vorzugsweise zwischen 0,5 und 90 Gew.% Wirkstoffmischungen. Sie können nach an sich bekannten Methoden durchgeführt werden.

Zur Herstellung von direkt versprühbaren Lösungen, Emulsionen, Pasten und Ölsdispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische oder aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon und Wasser, in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol, Nonylphenol, Pulver, Streu- und Stäubmittel können durch Mischen oder gemeinsames Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin-Sulfitablaugen und Methylcellulose in Betracht.

Vermahlen von herbizidem Wirkstoff und/oder Antidot mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Herstellungsbeispiele

Die in den nachstehenden Beispielen wiedergegebenen Vorschriften wurden unter entsprechender Abwandlung der Ausgangsverbindungen zur Gewinnung weiterer Verbindungen der Formeln Ia und Ib benutzt; die erhaltenen Verbindungen sind in den nachfolgenden Tabellen 1 und 2 mit physikalischen Angaben aufgeführt.

Beispiel 1

1-(2-Chlorphenyl)-2-(4-fluorphenyl)-3-(1,3,4-triazol-1-yl)aminopropan

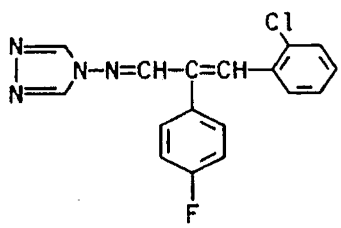

Eine Lösung aus 260,5 g 3(2-Chlorphenyl)-2-(4-fluorphenyl)propenal in 1500 ml Toluol wurde bei 25° C mit 168 g N-Amino-1,3,4-triazol versetzt und das so erhaltene Reaktionsgemisch 48 Std. unter Rückfluß erhitzt. Zur Aufarbeitung wurde das erhaltene Reaktionsgemisch mit Wasser versetzt, wobei das Produkt als Festkörper anfiel.

Ausbeute 179 g (55 %); Fp. 198 - 200° C; Wirkstoffbeispiel 1.001

Tabelle 1

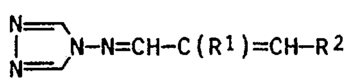

Ia

| Wirkstoff Nr. | $R^1$ | $R^2$ | phys. Daten Fp. (°C); $^1$H-NMR (δ in ppm) |
|---|---|---|---|
| 1.001 | 4-F-$C_6H_4$ | 2-Cl-$C_6H_4$ | 198 - 200 |
| 1.002 | $C_6H_5$ | 2-Cl-$C_6H_4$ | 180 - 182 |

Tabelle 2

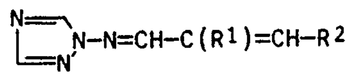

Ib

| Wirkstoff Nr. | $R^1$ | $R^2$ | phys. Daten Fp. (°C); $^1H$-NMR (δ in ppm) |
|---|---|---|---|
| 2.001 | $4-F-C_6H_4$ | $2-Cl-C_6H_4$ | 153 - 155 |
| 2.002 | $C_6H_5$ | $2-Cl-C_6H_4$ | 96 - 101 |

Anwendungsbeispiele

Der Einfluß verschiedener Vertreter der erfindungsgemäßen herbiziden Mittel bzw. Mittelkombinationen, bestehend aus Herbizid und synergistisch wirkender Verbindung, auf das Wachstum von erwünschten und unerwünschten Pflanzen im Vergleich zum herbiziden Wirkstoff allein wird durch die folgenden biologischen Beispiele aus Gewächshausversuchen belegt:

Zur Anzucht der Testpflanzen dienten Plastikblumentöpfe mit rund 300 $cm^3$ Inhalt und lehmiger Sand mit etwa 3,0 % Humus als Substrat. Die Samen der Testpflanzen wurden nach Arten getrennt, flach eingesät und befeuchtet. Danach deckte man die Gefäße mit durchsichtigen Plastikhauben ab, bis die Samen gleichmäßig gekeimt und die Pflanzen angewachsen waren.

Für die Nachauflaufbehandlung wurden die Testpflanzen je nach Wuchsform erst bis zu einer Wuchshöhe von 3 bis 20 cm angezogen und dann behandelt. Die herbiziden Mittel wurden hierbei in Wasser als Verteilungsmittel suspendiert oder emulgiert und mittels fein verteilender Düsen gespritzt.

Als Benzothiadiazinderivat II wurde in den biologischen Beispielen II.005 verwendet

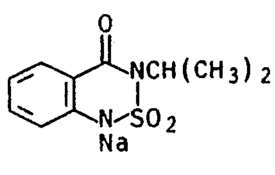

II.005

Der herbizide Wirkstoff II.005 wurde als kommerziell formuliertes Produkt (480 g/l Emulsionskonzentrat) appliziert.

Sämtliche synergistisch wirkende Verbindungen wurden mit 10 Gew.-% Wirkstoff in einer Mischung aus 70 % Lösungsmittel, 20 % Emulgator und 10 % Tensid aufbereitet.

Sofern das Herbizid und der Synergist gemeinsam appliziert wurden, waren die vorstehend beschriebenen Einzelformulierungen vorher gemischt worden.

Die Versuchsgefäße wurden im Gewächshaus aufgestellt, wobei für wärmeliebende Arten 18 bis 35 °C und für solche gemäßigtere Klimate 10 bis 25 °C bevorzugt wurden.

Die Versuchsperiode erstreckte sich über 3 bis 5 Wochen. Während dieser Zeit wurden die Pflanzen gepflegt, und ihre Reaktion auf die einzelnen Behandlungen wurde erfaßt. Bewertet wurde die Schädigung durch die chemischen Mittel anhand einer Skala von 0 % bis 100 % im Vergleich zu den unbehandelten Kontrollpflanzen. Dabei bedeutet 0 keine Schädigung und 100 eine völlige Zerstörung der Pflanzen.

In den folgenden Beispielen wird die Wirkung der erfindungsgemäß verwendbaren Mittel gezeigt, ohne die Möglichkeit weiterer Anwendungen auszuschließen.

Bei diesen Beispielen wurde nach der Methode von S.R. Colby (Weeds 15, 20) derjenige Wert E errechnet, der bei einer nur additiven Wirkung der Einzelwirkstoffe zu erwarten ist.

Die Berechnung erfolgt nach

$$E = X + Y - \frac{XY}{100}$$

wobei
X = Prozentsatz Wirkung mit Präparat A bei einer Aufwandmenge a
Y = Prozentsatz Wirkung mit Präparat B bei einer Aufwandmenge b
E = zu erwartende Wirkung (in %) durch A+B bei den Aufwandmengen a+b bedeuten.

Ist der beobachtete Wert höher als der nach Colby errechnete Wert E, so liegt eine synergistische Wirkung vor.

Die anschließenden Tabellen dokumentieren die synergistische Wirkung der Beispielverbindung 1.001, durch das die herbizide Aktivität des Wirkstoffs II.005 gegen eine unerwünschte Pflanze erheblich verbessert wird, ohne daß die Kulturpflanze geschädigt wird.

Tabelle 3

| Synergistische Aktivität der erfindungsgemäßen Verbindung 1.001 bei gemeinsamer Anwendung mit dem herbiziden Wirkstoff II.005 | | | |
|---|---|---|---|
| Aufwandmenge [kg/ha] | | Amaranthus retroflexus | |
| Beispiel Nr. 1.001 | Herbizid II.005 | Schädigung [in %] | E [nach Colby] |
| - | 1,25 | 55 | - |
| - | 0,625 | 45 | - |
| - | 0,313 | 35 | - |
| 0,25 | - | 0 | - |
| 0,25 | 1,25 | 100 | 55 |
| 0,25 | 0,625 | 90 | 45 |
| 0,25 | 0,313 | 88 | 35 |

Tabelle 4

| Synergistische Aktivität der erfindungsgemäßen Verbindung 1.001 bei gemeinsamer Anwendung mit dem herbiziden Wirkstoff II.005* | | | | | |
|---|---|---|---|---|---|
| Aufwandmenge [kg/ha] | | Testpflanzen [Schädigung in %; E nach Colby] | | | |
| Beispiel Nr. 1001 | Herbizid II.005 | Glycine max. | E | Amaranthus retroflexus | E |
| - | 1,25 | 0 | - | 95 | - |
| - | 0,325 | 0 | - | 58 | - |
| - | 0,313 | 0 | - | 35 | - |
| - | 0,156 | 0 | - | 20 | - |
| 0,125 | - | 0 | - | 0 | - |
| 0,125 | 1,25 | 0 | 0 | 100 | 95 |
| 0,125 | 0,625 | 0 | 0 | 100 | 58 |
| 0,125 | 0,313 | 0 | 0 | 98 | 35 |
| 0,125 | 0,156 | 0 | 0 | 85 | 20 |

* zusätzlich enthielten alle Spritzungen 2,8 1.ha Spritölkonzentrat als oberflächenaktiven Zusatzstoff.

## Ansprüche

1. N-Aminotriazolderivate der allgemeinen Formel Ia und Ib,

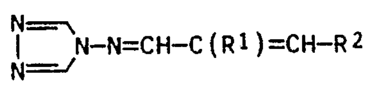

Ia

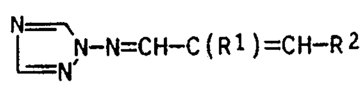

Ib

in der die Substituenten folgende Bedeutung haben:
$R^1$, $R^2$ $C_1$-$C_8$-Alkyl; $C_3$-$C_8$-Cycloalkyl; $C_5$-$C_8$-Cycloalkenyl; Tetrahydropyranyl; Norbornyl; Phenyl, Biphenyl, Naphthyl oder Pyridyl, wobei die aromatischen Reste ein bis fünf Halogenatome und/oder ein bis drei der folgenden Gruppen tragen können: Hydroxy, Phenoxy, Nitro, Amino, Cyano, $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio
sowie deren Umwelt- und Kulturpflanzen-verträgliche Salze.

2. N-Aminotriazolderivate der allgemeinen Formel Ia und Ib gemäß Anspruch 1, in der die Substituenten $R^1$ und $R^2$ folgende Bedeutung haben: Phenyl, welches ein bis drei Halogenatome und/oder ein bis zwei der folgenden Reste tragen kann: Methyl, Difluormethyl, Trifluormethyl, Methoxy, Difluormethoxy, Trifluormethoxy und/oder Methylthio.

3. Herbizide Mittel, enthaltend neben inerten Zusatzstoffen mindestens ein N-Aminotriazolderivat der Formel Ia und/oder Ib gemäß Anspruch 1 sowie mindestens einen herbiziden Wirkstoff aus der Gruppe der Benzothiadiazinderivate der Formel II

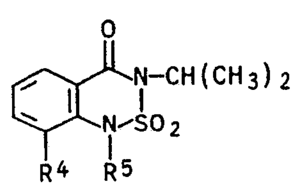

II

in der die Substituenten folgende Bedeutung haben:

$R^4$ Wasserstoff; Halogen; $C_1$-$C_4$-Alkyl oder $C_1$-$C_4$-Alkoxy und
$R^5$ Wasserstoff oder Cyano
oder dessen Umwelt- und Kulturpflanzen-verträgliche Salze.

4. Herbizide Mittel nach Anspruch 3, enthaltend mindestens ein N-Aminotriazolderivat der Formel Ia und/oder Ib gemäß Anspruch 1 sowie als herbiziden Wirkstoff 3-Isopropyl-1H-2,1,3-benzothiadiazin-4(3H)-2,2-dioxid oder dessen umweltverträgliche Salze.

5. Herbizide Mittel nach Anspruch 3, enthaltend ein N-Aminotriazolderivat Ia und/oder Ib gemäß Anspruch 1 und ein Benzothiadiazin II im Gewichtsverhältnis von 4:1 bis 0,01:1.

6. Verfahren zur selektiven Bekämpfung von unerwünschten Pflanzenwuchs, dadurch gekennzeichnet, daß man ein N-Aminotriazolderivat der Formel Ia und/oder Ib gemäß Anspruch 1 und ein Benzothiadiazin II gemäß Anspruch 3 vor, bei oder nach der Aussaat der Kulturpflanzen, vor oder während des Auflaufens der Kulturpflanzen gleichzeitig oder nacheinander ausbringt.

7. Herbizide Mittel, enthaltend mindestens ein N-Aminotriazolderivat der Formel Ia und/oder Ib gemäß Anspruch 2 sowie mindestens einen herbiziden Wirkstoff aus der Gruppe der Benzothiadiazine II gemäß Anspruch 3 und inerte Zusatzstoffe.

8. Verfahren zur selektiven Bekämpfung von unerwünschtem Pflanzenwuchs, dadurch gekennzeichnet, daß man ein N-Aminotriazolderivat der Formel Ia und/oder Ib gemäß Anspruch 2 und ein Benzothiadiazin II gemäß Anspruch 3 vor, bei oder nach der Aussaat der Kulturpflanzen, vor oder während des Auflaufens der Kulturpflanzen gleichzeitig oder nacheinander ausbringt.